# EUROPEAN PATENT APPLICATION

(11) **EP 2 738 703 A2**
(43) Date of publication of application: **04.06.2014**
(21) Application number: 13194904.2
(22) Date of filing: 28.11.2013
(51) Int. Cl.: G06F 19/00

(54) **Data visualization method and apparatus utilizing receiver operating characteristic analysis**

(30) Priority: 30.11.2012 US 201261732039 P; 26.11.2013 US 201314090382
(71) Applicant: Kabushiki Kaisha TOPCON, Tokyo 174-8580 (JP)
(72) Inventor: Reisman, Charles A., Mamaroneck, NY 10543 (US)
(74) Representative: Tischner, Oliver

(57) **Abstract**

A system and method for presenting experimentally-determined data relating to a region of interest on a medical patient to a user. A computer memory stores a ROC analysis of historical data for a region of interest of a plurality of different subjects. A computer processor is programmed to compare the experimentally-determined data to historical data based on the ROC analysis of the historical data and identify the experimentally-determined data to be utilized in reaching an at least preliminary conclusion regarding the medical patient. A display device is operatively connected to the computer processor to present a visible display graphically depicting a result of the comparison performed by the processor.

## Description

### CROSS-REFERENCE TO RELATED APPLICATIONS

This application claims the benefit of U.S. Provisional Application No. 61/732,039, filed November 30, 2012, which is incorporated in its entirety herein by reference.

### BACKGROUND OF THE INVENTION

### 1. Field of the Invention

This application relates generally to optical diagnostic methods and apparatus and, more specifically, to a data visualization method and computer apparatus for graphically depicting a result of an optical or other type of scan of a biological tissue.

### 2. Description of Related Art

Conventional diagnostic scans typically generate significant amounts of data that must be reviewed by a clinician to make an accurate diagnosis. Retinal thickness can be measured at several locations within the human eye as one of several tests performed to accurately diagnose a subject with glaucoma, for example. However, the retina may atrophy at different rates at different locations, and for different people, causing local instances of retinal thinning that vary by location and patient while other portions of the retina remain unchanged. In addition, specific tissues or features may be located at slightly different locations over a population of individuals. Accordingly, such measurements must be conducted for a significant number of locations within the eye to thoroughly assess the condition of the retina for each individual patient.

Additionally, conditions such as glaucoma can manifest themselves in a variety of different ways. Retinal thickness is one factor to consider as part of an effort to determine whether a subject has glaucoma because representative patterns of tissue damage or atrophy may be observable. Because the subject's retinal thickness does not, by itself, definitively distinguish those with glaucoma from those without, additional tests must also be conducted, further adding to the amount of data that must be examined for an accurate diagnosis to be made.

Evaluating such a large volume of data is complex and time consuming. Unreliable data will also often be included in data being examined for diagnostic purposes. It is impractical to review historical examination data as part of a diagnostic process in an effort to determine what data, if any, may be unreliable.

### BRIEF SUMMARY OF THE INVENTION

Accordingly, there is a need in the art for a method and apparatus for providing large quantities of data in a condensed format, and optionally for providing an indication whether such large quantities of data include a subset data that may be considered more reliable than another subset of data.
In view of the above, the system according to claim 1 and the method according to claim 8 are provided.

Since conditions such as glaucoma can be difficult to diagnose in its early stages, even glaucoma specialists can disagree about whether a new patient with a certain cluster of symptoms has glaucoma. Reaching a proper diagnosis can be facilitated by comparing data from an optical coherence tomography ("OCT") scan, for example, conducted for a region of interest within the eye of the specific patient in question to analogous, historical data from a pool of normative subjects on a location-by-location basis. The method and apparatus disclosed herein can present the data from the OCT scan for the specific patient and the historical data in a manner for ready comparison. Additionally, the method and apparatus can present the historical data in a manner that reveals a pattern in which a symptom has historically manifested itself to indicate the region of interest that can be a reliable predictor of glaucoma or other condition.

According to another aspect, the present disclosure involves a method of presenting experimentally-determined data relating to a region of interest on a medical patient for predicting whether the medical patient has a medical condition. Such a method includes, using a computer processor, accessing a ROC analysis of historical data for the region of interest on each of a plurality of different subjects from a non-transitory computer memory. Each of the plurality of different subjects is known as having the medical condition or not having the medical condition when the historical data was collected, and the region of interest on each of the plurality of different subjects corresponds to the region of interest on the medical patient. A subset of spatial locations is identified in the region of interest where the experimentally-determined data is an accurate predictor of the medical condition to be utilized to reach an at least preliminary conclusion regarding the medical patient based on the ROC analysis of the historical data. The experimentally-determined data at the subset of spatial locations of the medical patient is compared to the ROC analysis of the historical data stored by the computer-readable medium. A graphic display is generated to be presented by a display device operatively connected to the computer processor to a user for graphically expressing a result of said comparing.

According to another aspect, the present disclosure involves a method of predicting whether a medical patient comprises a medical condition. Utilizing a computer processor, a measured thickness of tissue at a plurality of spatial locations on an eye of the medical patient is received. A ROC analysis of historical tissue thickness data for eyes belonging to a plurality of different subjects that were known as having the medical condition or not having the medical condition when the historical tissue thickness data was collected is used to identify a subset of the spatial locations where the measured thickness is an accurate predictor of the medical condition. The measured thickness at each location in the subset of the spatial locations is compared to threshold thickness values for each location in the subset of the spatial locations. The threshold thickness values are used to preliminarily distinguish between medical patients with the medical condition and medical patients without the medical condition. An image that graphically represents a result of said comparing and a likelihood that each spatial location within the subset of the spatial locations provides an accurate prediction of whether the medical patient is afflicted with the medical condition is generated to be presented to a user for rendering a preliminary diagnosis.

The above summary presents a simplified summary in order to provide a basic understanding of some aspects of the systems and/or methods discussed herein. This summary is not an extensive overview of the systems and/or methods discussed herein. It is not intended to identify key/critical elements or to delineate the scope of such systems and/or methods. Its sole purpose is to present some concepts in a simplified form as a prelude to the more detailed description that is presented later.

### BRIEF DESCRIPTION OF SEVERAL VIEWS OF THE DRAWING

The invention may take physical form in certain parts and arrangement of parts, embodiments of which will be described in detail in this specification and illustrated in the accompanying drawings which form a part hereof and wherein:
FIG. 1 shows an illustrative embodiment of an OCT system for measuring a quality of an eye;
FIG. 2 shows a projection of a sectional view of the eye shown in FIG. 1 view taken along line 2-2, with a grid defining different spatial locations in a region of interest on an inner surface of the eye;
FIG. 3 shows an illustrative embodiment of an array comprising entries that each correspond to a different one of the spatial locations defined by the grid shown in FIG. 2;
FIG. 4 shows an illustrative embodiment of a thickness array relating a measured retinal thicknesses to different locations within a region of interest in the eye;
FIG. 5 shows an illustrative embodiment of a ROC array that provides an indication about the predictive nature of data collected at various different spatial locations in a region of interest within an eye; and
FIG. 6 shows an illustrative embodiment of a color-coded, non-textual presentation of data included in a ROC array.

### DETAILED DESCRIPTION OF THE INVENTION

Certain terminology is used herein for convenience only and is not to be taken as a limitation on the present invention. Relative language used herein is best understood with reference to the drawings, in which like numerals are used to identify like or similar items. Further, in the drawings, certain features may be shown in somewhat schematic form.

It is also to be noted that the phrase "at least one of", if used herein, followed by a plurality of members herein means one of the members, or a combination of more than one of the members. For example, the phrase "at least one of a first widget and a second widget" means in the present application: the first widget, the second widget, or the first widget and the second widget. Likewise, "at least one of a first widget, a second widget and a third widget" means in the present application: the first widget, the second widget, the third widget, the first widget and the second widget, the first widget and the third widget, the second widget and the third widget, or the first widget and the second widget and the third widget.

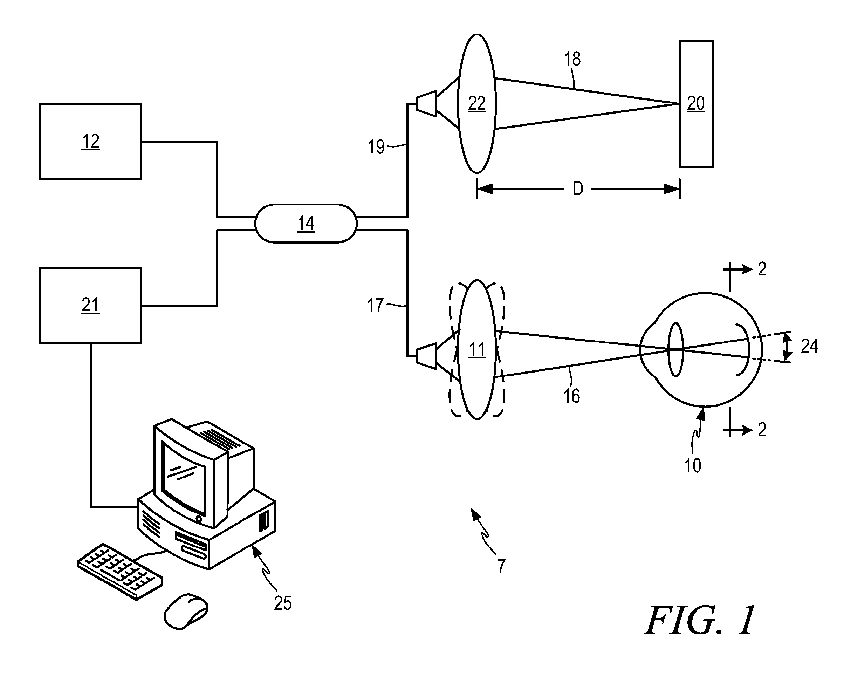

FIG. 1 shows an illustrative embodiment of a system 7 for performing a test on a subject as part of a medical examination. As shown, the system 7 is an optical coherence tomography ("OCT") system that performs an imaging technique used in ophthalmology to generate cross-sectional images of portions of the eye 10, such as the retina (or more specifically, the nerve fiber layer, ganglion cell layer ("GCL"), ganglion cell plus inner plexiform layer (GCL+), and nerve fiber layer plus ganglion cell layer plus inner plexiform layer (a.k.a., ganglion cell complex or GCC), for example. Although the present disclosure uses the OCT system to measure retinal thickness as an illustrative example of the system 7 for the sake of clearly describing the subject method and apparatus, it is to be understood that the present disclosure is not so limited. The system 7 can include any imaging or other such diagnostic apparatus that generates data relating to different spatial locations of a subject as part of a medical examination. For example, the system 7 can include a computer axial tomography ("CT") modality, a magnetic resonance imaging ("MRI") modality, an ultrasound modality, etc... to capture data concerning different portions of a subject's body, such as internal organs, muscles, nerves, blood vessels, bones, etc... without departing from the scope of this disclosure.

Generally, OCT involves impinging light from a low-coherence broadband light source 12 onto portions of the eye 10, and observing reflected light to generate a cross-sectional image of those portions of the eye 10. Light from the light source 12 is split by an optical adaptor 14 such as a beam splitter, fiber coupler or the like, into two portions: a sample portion 16 that travels along a path 17 toward the eye 10, and a reference portion 18 that travels along a path 19 toward a reference reflector such as a mirror 20. The sample portion 16 and the reference portion 18 are at least partially reflected, and the reflected portions combined by the optical adaptor 14 and the intensity or other quality (e.g., wavelength, fraction of light reflected, etc...) of the combined, reflected light is sensed by a detector 21 operatively connected to transmit a signal indicative of the sensed quality to be received by a computer 25. When the distance travelled by the sample portion 16 is within a coherence length of the distance travelled by the reference portion 18, an optical interference pattern is created, affecting the intensity of the reflected and combined light. The intensity of the combined, reflected light varies depending upon the properties (e.g., tissue backscattering, polarization, etc...) of the portions of the eye 10 that are illuminated by the sample portion 16. Information about such properties of the illuminated portion of the eye 10 can then be determined based on the intensity of the combined, reflected light, and used to generate image data representing, or otherwise pertaining to, that illuminated portion of the eye 10.

The depth to which the sample portion 16 penetrates the eye 10 can be controlled in the time domain by varying a distance D separating a transmitter 22 of the reference portion 18 from the mirror 20. Alternatively, the depth of penetration can be controlled in the frequency domain by establishing an appropriate distance D, which can be fixed or variable to counterbalance the specific eye shape of each individual patient and/or saccadic eye movements for example, and utilizing a broadband light source or alternatively sweeping (e.g., repeatedly sequentially varying the frequency over a period of time) the wavelength of the light source 12 over a range of frequencies. Conducting a Fourier analysis on the combined, reflected light relates the light reflected at different frequencies to light reflected at different depths of the eye 10.

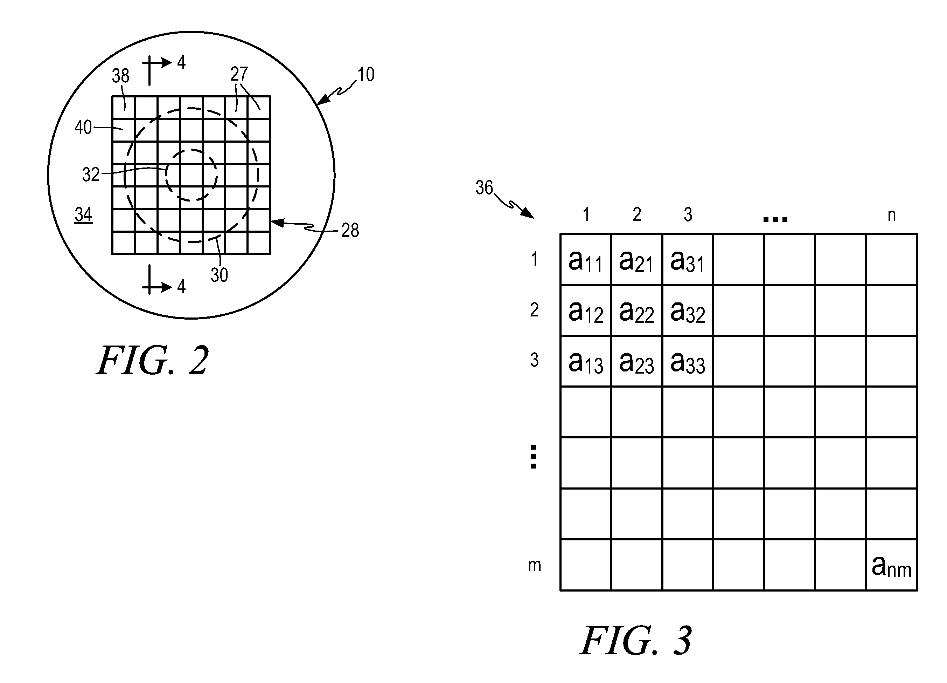

The sample portion 16 having a fixed penetration depth can be reflected through adjustment of a transmitter 11, such as a mirror, lens, etc..., for example, to various different orientations (illustrated using broken lines in FIG. 1) to illuminate different spatial locations 27 (FIG. 2) over a region of interest 24 (e.g., macula, optic disc, etc...) within the eye 10. FIG. 2 shows a projection of a sectional view of the eye 10 taken along line 2-2 in FIG. 1 onto a flat plane, with a grid 28 superimposed over the region of interest 24 as an example of how the different spatial locations 27 can be arranged within the region of interest 24. Although the projection of the eye 10 in FIG. 2 includes an inward-facing surface 34 at the posterior of the eye 10, the iris 30 and pupil 32 are shown in broken lines to provide a frame of reference for the view into the eye 10 appearing in FIG. 2. In other words, the inward-facing surface 34 at the posterior of the eye 10 is viewed by observing that surface 34 looking through the front of the eye 10. The grid 28 can include dimensions (e.g., a number of columns and rows, a width and height, etc...) suitable to overlay the region of the surface 34 of interest for examination purposes, and can be smaller than the projection as shown in FIG. 2, larger than the projection, or otherwise scaled as desired relative to the projection. Further, the grid 28 is not necessarily projected by the system 7 onto the region of interest 24 within the eye, but is shown in FIG. 2 simply to provide an example of how the different spatial locations 27 can be arranged.

Determining the intensity of light reflected by a spatial location 27 located within the region of interest 24 is referred to herein as an "A-scan". An A-scan involves detecting the intensity of reflected light corresponding to features within the eye 10 arranged in the axial direction of the sample portion 16 (FIG. 1) of light extending into the specific locations 27 (i.e., in the depth direction of the eye 10). The information conveyed by the reflected portion of light provides an indication of the structures encountered by the sample portion 16 of light along the axial direction for that spatial location 27. This scan is repeated for other spatial locations 27 within the region of interest 24, which can be another spatial location 27 in the same vertical column of the grid 28. The information obtained from the A-scans performed at the spatial locations 27 can be combined to collectively form a cross-sectional image of the region of interest 24, which is referred to herein as a "B-scan". A plurality of B-scans can also be combined to create a three-dimensional volume. For ophthalmic purposes, an illustrative example of the region of interest 24 for the A-scans can include a region extending from the cornea to the retina, and a B-scan can include a region extending from a medial border to a lateral border of the eye 10 and from the cornea to the retina. However, alternate embodiments can utilize data forming A-scans in regions of interest 24 that are shorter, spanning approximately 2-3 mm in length relative to the overall length of the eye 10, which can be approximately 25 mm for most patients.

The data of A-scans and B-Scans are two illustrative examples of the types of data that can be analyzed as described herein for diagnostic purposes, but do not constitute an exhaustive list of such data. For example, another type of data can include data collectively forming a so-called C-scan. Imaging devices such as charge-coupled device (CCD) cameras, for example, have been utilized to capture an *en face* image of the region of interest within the eye 10 utilizing a full-field illumination of that region. The resulting C-scan is formed from data corresponding to regions of interest arranged in a two-dimensional plane such as that appearing in FIG. 2, and eliminates the need for the electromechanical lateral scan procedures that combine data collected as part of separate, successive scans as described above. Adjusting the reference mirror stepwise and recording successive *en face* images also allows a three-dimensional representation of the region of interest to be created.

There are also many non-OCT systems (e.g., scanning laser ophthalmoscopes) that produce C-scans and possibly other collections of data to be used in a medical examination of an eye 10 that can be analyzed as described herein. Further, the data visualization techniques herein can also optionally be applied to evaluate and provide a graphical representation of such other data indicative of a characteristic such as signal intensity, for example, instead of a layer thickness. However, for the sake of brevity and to clearly describe the present data visualization method and apparatus, the representation and analysis of A-scan data, B-scan data, or a combination thereof, collected via OCT technology is used as an illustrative example.

The A-scan, B-scan, or a combination thereof, can be analyzed to determine a physical dimension or other quantification of the tissue forming the region of interest 24. Referring to the example of the OCT system used in ophthalmology once again, the thicknesses (in the axial direction of the sample portion 16) of the inner retinal layers of the eye 10 can be measured using either or both of the A-scan and the B-scan. The difference between the boundaries of different inner retinal layers appearing in the A-scan(s) or B-scan can be utilized to determine the thickness of the inner retinal layers, for example.

The intensity of the combined, reflected light (or other quality) sensed by the detector 21 is transmitted to the computer 25 shown in FIG. 1 for each different spatial location 27 (FIG. 2) in a first layer of the region of interest 24. The computer 25, which includes a processor for executing computer-executable instructions stored by a non-transitory, computer-readable medium such as a hard drive, for example, stores the sensed intensities in a database also stored on the hard drive. The database can be structured to store each value of the intensity sensed by the detector 21 with a link or other relationship to the respective different spatial location 27 in the region of interest 24 from which the corresponding light was reflected. For example, the intensity values of light reflected from each of the different spatial locations 27 can be stored in an array with entries corresponding to the grid 28. FIG. 3 shows an illustrative embodiment of an array 36 comprising entries that each correspond to a respective different one of the spatial locations 27 defined by the grid 28 shown in FIG. 2. In other words, the array 36 is an n x m array, having n columns and m rows, where n and m can be independently selected as any desired integer. According to the present embodiment, the grid 28 can also optionally be divided up into n columns and m rows, the intersections of which establish the different spatial locations 27. The cells of the array 36 can be arranged to store an intensity value of light reflected from a corresponding spatial location 27 with the same arrangement in the grid 28. For example, the intensity value of light reflected from the spatial location 38 can be stored as the value a₁₁ in FIG. 3. Likewise, the intensity value of light reflected from the spatial location 40 can be stored as the value a₁₂, and so on. Thus, the array can represent the intensity values of the A-scan for a given tissue depth.

Similarly, the intensity values of light reflected from the different spatial locations 27, but for a different axial penetration depth of the sample portion 16 of the light into the region of interest 24, can be stored in a separate array. This separate array can optionally be analogous to the array 36, with each cell storing the intensity value corresponding to a similarly-arranged cell in the grid 28. A different array can be provided to store the intensity values from the different spatial locations 27 for each A-scan. The data in the plurality of arrays can collectively be utilized by the computer 25 to generate the B-scan and determine the layer thickness at the different spatial locations 27.

Although the data indicative of the intensity values is described above as being stored in groupings corresponding to C-scans (two-dimensional planes at a predetermined depth within the eye 10), such a grouping is arbitrary. The data utilized by the data visualization method and apparatus herein can be stored in a non-transitory, computer readable medium in any desired grouping, or even randomly.

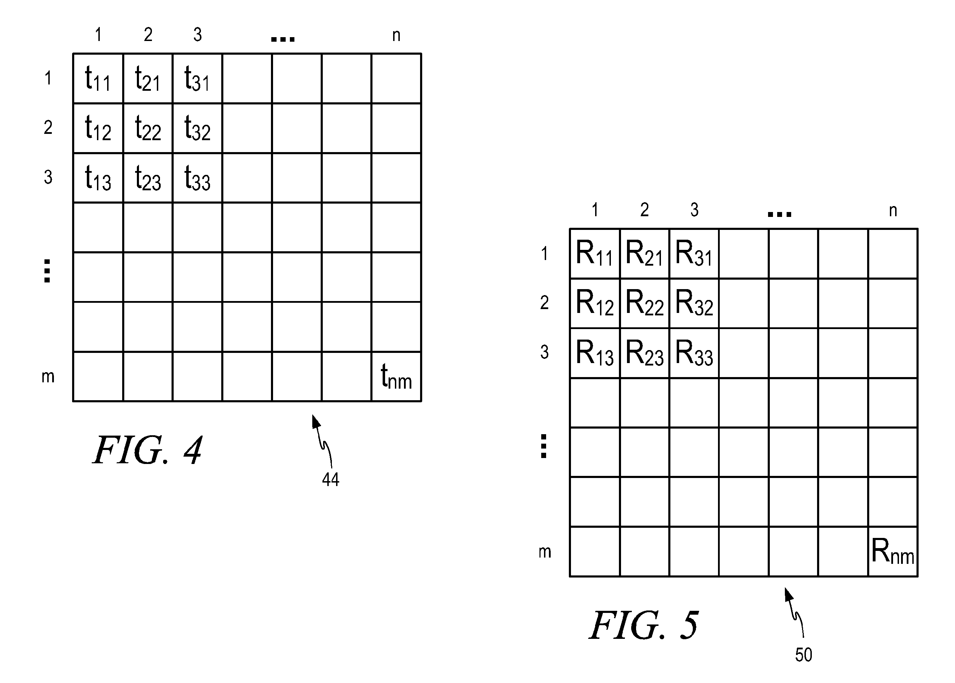

The layer thickness calculated at each of the different spatial locations 27 determined from the B-scan can be stored and arranged in a thickness array 44 as shown in FIG. 4. Again, the thickness values t₁₁ to tₙₘ stored in the cells of the array 44 can correspond to the layer thickness determined at each of the similarly-arranged spatial locations 27 of the grid 28 (FIG. 2). In other words, the thickness value t₁₁ corresponds to the layer thickness at the spatial location 38 (FIG. 2), while the thickness value t₁₂ corresponds to the layer thickness at spatial location 40 (FIG. 2).

The computer 25 can also optionally store, or at least access over a communication network, data pertaining to a receiver-operating-characteristic ("ROC") analysis, such as the ROC array 50, shown in FIG. 5. The ROC array 50 includes data generated as a result of a ROC analysis conducted on a plurality of individual thickness arrays that are analogous to the thickness array 44 described above, but populated with data from different subjects, each independently verified as either: suffering from a specific disease, ailment, fault or condition; or not suffering from the specific disease, ailment, fault or condition. The disease, ailment, fault or other condition will be referred to generally below as a "condition."

There are but two possible outcomes of a medical examination to determine if a subject has a specific condition: either the subject has the condition, or the subject does not. For ophthalmic purposes, subjects suffering from conditions such as glaucoma or multiple sclerosis typically exhibit rough patterns of tissue atrophy, causing layer thicknesses of features within the eye to thin as the condition progresses. Test results for such conditions, however, will often depend on the degree or the extent of atrophy exhibited. As such, there may be no bright line cutoff layer thickness that definitively differentiates a subject with the condition from one without the condition. A layer thickness limit, identified in an effort to distinguish a subj ect with glaucoma, for example, from a subject without glaucoma will indeed correctly identify some subj ects who truly suffer from glaucoma. If the retinal thickness of a subject who actually has glaucoma is less than the layer thickness limit, a true positive ("TP") result is said to occur. Such a thickness limit will also correctly identify some subjects who, in reality, do not suffer from glaucoma based on their retinal thickness, which is considered a true negative ("TN") result.

However, since layer thickness is but one factor to consider in a glaucoma diagnosis, and not determinative by itself, the thickness limit will also cause some subjects who do not have glaucoma to be incorrectly diagnosed as having glaucoma. For such subjects their retinal thickness will be less than the layer thickness limit even though they do not suffer from glaucoma, causing a false positive ("FP") result to occur. Conversely, the thickness limit will also cause some subjects who do have glaucoma to be incorrectly diagnosed as not having glaucoma (i.e., the retinal thickness of such subjects is greater than the layer thickness limit even though these subjects do have glaucoma), causing a false negative ("FN") result to occur. Utilizing the data in the ROC array 50 to interpret the data in the thickness array 44 can improve the accuracy of a test for glaucoma based on the retinal thickness over that of a test based solely on a comparison of a measured retinal thickness to a threshold value alone.

The ROC values Rₙₘ populating the cells of the ROC array 50 in FIG. 5 are determined based on the data populating thickness arrays 44 for a plurality of different subj ects that are known to have a condition (glaucoma in the present example) and a plurality of subj ects that are known not to have the condition in question. The value of the retinal thickness for a given spatial location 27 (FIG. 2) in the region of interest 24 as established by the grid 28 is analyzed for each subject, in view of the subject's actual condition to determine whether the retinal thickness value causes a hypothetical result of TP, TN, FP or FN to occur for glaucoma. Table 1 illustrates an example of the retinal thickness value a₁₁ (FIG. 4) arranged in a cell (1,1) of the thickness array 44 for a plurality of different subjects (subjects 1-10). This retinal thickness is measured at the spatial location 38 (FIG. 2) in the region of interest 24 in the eye 10 of each subject. A layer thickness limit of one hundred (100 µm) is assumed as the cutoff differentiating subjects with glaucoma from those without glaucoma for the present example. Subjects are believed to have glaucoma if their retinal thickness < 100 µm, and are believed to not have glaucoma if their retinal thickness is ≥ 100 µm). Whether each subject actually suffers from glaucoma (i.e., "Actual Result" = Y if the subject has been independently determined to actually have glaucoma, and N if the subject has been independently determined to actually not have glaucoma) and the hypothetical result of the test based on the retinal thickness, alone, for each subj ect are also tabulated.

**Table 1. Retinal thickness at a common spatial location for different subjects.**

| Subject No. | Retinal Thickness (a₁₁) (µm) | Actual Result | Hypothetical Result |
|---|---|---|---|
| 1 | 85 | Y | TP |
| 2 | 200 | N | TN |
| 3 | 110 | Y | FN |
| 4 | 81 | N | FP |
| 5 | 177 | N | TN |
| 6 | 76 | Y | TP |
| 7 | 128 | N | TN |
| 8 | 160 | N | TN |
| 9 | 42 | N | FP |
| 10 | 127 | N | TN |

To improve the relevance of the data contained in the ROC array 50, the plurality of different subjects whose data are to be considered can be filtered to include only a desired demographic. To act as an aid in diagnosing subjects with early-stage glaucoma, for example, the data populating Table 1 can be limited to that corresponding to subjects with early-stage glaucoma. For example, both the appearance of the optic disc as well as visual field test measurements are commonly used to diagnose a subject with glaucoma. The optic disc evaluation is somewhat subjective, but the visual field results are objective and are reported as a mean deviation ("MD") in decibel scale. An MD between 0 to -6dB, for example, (or perhaps better than -6dB without an upper bound) can be considered "early" glaucoma, between -6dB and -12dB, for example, can be considered "moderate" glaucoma, and below -12dB, for example, can be "advanced" or "severe" glaucoma. Of course, these ranges of the MD are illustrative, and can be varied as desired to distinguish between conditions. According to alternate embodiments, the moderate and advanced groups can be combined into a single grouping. Yet other embodiments can divide the early stage glaucoma group into two parts: -3 to -6dB assigned to "early" whereas better than -3dB is "very early" (or perhaps even just a suspected glaucoma patient, but not yet a patient). Likewise, the subjects can be filtered based on age, other independently verified conditions, or any combination thereof.

According to alternate embodiments, the data of the different subjects populating the thickness arrays 44 used to calculate the values for the ROC array 50 can be normalized before the ROC calculations are performed. As part of this normalization, the retinal thickness variance amongst the subjects who do not have glaucoma will be minimized to increase ROC values Rₙₘ and thereby improve the ability of the ROC array 44 to identify specific locations 27 in the eye 10 that best discriminate between subjects with glaucoma from those without. For example, normalization of the data used to generate the ROC array 50 based on a physical feature of the eye of each subject before performing the ROC calculations. The retinal thickness data of each subject can be normalized based on the size and/or position of at least one of: the optic disc, fovea, blood vessels, nerve fiber layer arcuates, other portion of the eye, and any combination thereof. According to alternate embodiments, the specific locations 27 (FIG. 2) corresponding to the thickness data included in the thickness array 44 (FIG. 4) can be transformed/normalized based on the size and/or position of landmark features. For example, normalization can be performed based on the size of the annular-shaped ganglion cell layer ("GCL") formation in the macular region. A spatial transformation specific to each different subject could be performed such that the specific locations 27 where the A-scans are performed for each subject are located at approximately the same position relative to the annular GCL formation. Other types of normalization that can be performed on each subject's data before that data is utilized as part of the ROC calculations can be based on the angle between the fovea and optic disc, the radius of the ganglion cell layer and the inner plexiform layer in the macula, etc... Information such as angles and radius measurements, for example, used to perform the ROC calculations may also benefit from being converted into a different coordinate system (e.g., Cartesian coordinates to polar coordinates). Another embodiment of the normalization that can be performed requires the amplitude of the thickness (or other) measurements by the OCT scan to be adjusted based on the average amplitude calculated over the image, or a sub-region of the image (which can be relative to an identified landmark or landmarks). Such a normalization process may be based on Pattern Standard Deviation ("PSD") calculations.

The thickness arrays 44 for the plurality of different subjects can optionally be compiled from OCT scans performed on the subject's right eye or a left eye. However, the thickness arrays 44 corresponding to data collected from a left eye of the different subjects can optionally be used to generate a ROC array 50 to be used specifically evaluating the OCT scan of current subject's left eye. A right-eye-specific ROC array 50 can also optionally be generated for comparison purposes when evaluating an OCT scan of a current patient's right eye.

According to alternate embodiments, the data collected in the thickness array 44 for a left eye of the plurality of different subj ects can optionally be reflected about a vertical axis to create a mirror image of that array. The reflected array can then be used along with thickness arrays 44 populated with data collected from the right eye of the plurality of different subjects to generate the ROC array 50.

The values in the array 36, each of the thickness arrays 44, or a combination thereof can correspond to desired spatial locations 27 having a predetermined relationship to a landmark location in the respective subjects' eyes according to other embodiments. The desired spatial locations 27 can be centered on a per-scan basis based on some identified landmark within the scan. For example, the array 36 and/or thickness arrays 44 can be translated in the n, m (i.e., X/Y dimensions), rotated about a desired cell or as a whole, in order to place the optic disc, fovea or other structural feature within a pre-specified location in the array space. Such operations pose other opportunities to minimize variance that may otherwise occur. According to alternate embodiments, ocular measurements such as axial eye length, for example, may also be used to calibrate or fine-tune the actual scan dimensions or scan location. In other words, the scan dimensions can cover a specific proportion of each eye based on the specific size of each eye be scanned.

According to yet other embodiments, the specific operations performed for normalization and transformation purposes can optionally be replaced by transforming the coordinates of the data included in the arrays. For instance, an Affine transform is an example of a transformation that can be performed on a scan-by-scan basis, optionally based on identified landmarks that might include the center of the optic disc, the fovea, or other features. The Affine transformation preserves straight lines (i.e., all points lying on a line initially still lie on a line after transformation) and ratios of distances between points lying on a straight line. However, non-linear transforms are also possible - on a scan-by-scan basis, possibly based on identified landmarks that might include the center of the optic disc, the fovea, or other features.

An example of a complete data set is shown populating Table 1. There are no subjects for whom a thickness measurement at the specific location 27 (FIG. 2) in question is missing. However, there may be instances where a data measurement is missing for a specific location for a given subj ect, or the value recorded may be such an outlier (e.g., an order of magnitude larger than other values) to be obviously erroneous. Such abnormal values can be factored into the ROC calculations differently than present, expected values. For example, if a thickness measurement for a given, specific location 27 in the eye 10 is routinely absent from the thickness array 44 of a substantial number of subj ects, the ROC calculations may not be performed for that given, specific location 27. According to alternate embodiments, the ROC calculations described herein may be performed for such a given, specific location, but such calculations can factor in the number of subj ects for whom a thickness measurement is available at that specific location 27. This sample size can be noted for subj ects who have been independently diagnosed with glaucoma and subjects who have been independently diagnosed as not having glaucoma. The sample size can optionally be used as a weighting factor to affect the ROC value, and thus, the usefulness of such value in diagnosing glaucoma.

Alternate embodiments may involve down sampling the thickness data, such that the size (i.e., integer selected for n and m) of the thickness array 44 can be smaller than (e.g., a subset) the size of an array populated by all data collected by an OCT scan. Down sampling can take any of various forms such as simple down sampling, which involves selecting one of many available data points to be included in the analysis, or performing an operation on clusters of data points such as averaging, or taking the median, quantile, etc... For instance, an OCT scan can result in an array having x columns and y rows. A value of at least one of x and y is less than a value of at least one of n and m, respectively. Additionally, the same can be said for the ROC array 50. The ROC array 50 can be smaller in size (i.e., fewer columns and/or rows) than the thickness arrays 44 storing data used to generate the ROC array 50. For such embodiments, the ROC array 50 can be generated for a subset of the data in the thickness arrays 44. According to alternate embodiments, the OCT scan image data can itself be downsampled. For such embodiments, the frequency at which OCT data is stored to form an A-scan and/or B-scan is collected can optionally be lower than the frequency at which the OCT system actually collects samples. For instance, depending on the level of detail required for analysis purposes, the frequency at which the OCT system actually collects samples can be downsampled by a factor of 2, meaning that only every other sample collected is stored for inclusion in the A-scan and/or B-scan. As another example, the OCT scan image data can optionally be downsampled by averaging collections or groupings of OCT data, calculating the median value or other notable value or arbitrary quantile that is representative of such collections and groupings without requiring every instance of the OCT scan data making up those collections and groupings to be individually and separately utilized.

Based on such information for the plurality of different subjects, optionally filtered, normalized or otherwise pre-processed, the true positive rate ("TPR") can be calculated as the ratio TP/(TP+FP), and the false positive rate ("FPR") can be calculated as the ratio FP/(TN+FN). The TPR (also known as the sensitivity) can be plotted against the FPR (1-specificity) to generate a ROC curve. The area under this ROC curve can be calculated and assigned as the value of R₁₁ in FIG. 5 corresponding to the specific location 38 (FIG. 2) of the region of interest 24 in the eye 10.

When the results of a test fall into one of two well-defined categories as in the previous example in Table 1, such as either the presence or absence of a disease, then the test has only one pair of sensitivity and specificity values. However, according to alternate embodiments, diagnostic scenarios commonly involve considerable variations in the confidence levels of a diagnosis amongst those who interpret an OCT scan or the like. Thus, two or more pairs of sensitivity and specificity values can be utilized to describe the full range of diagnostic performance of a test.

To deal with a plurality of pairs of sensitivity and specificity values, the sensitivities can be plotted as the y coordinates and the FPR (or 1-specificities) can be plotted as the x coordinates. Each discrete point on the plot is generated by using a different cutoff level for a positive test result. The ROC curve can be estimated from these discrete points, by making the assumption that the test results follow a binormal distribution. Again, the area under such a ROC curve can be calculated and assigned as the value of R₁₁ in the ROC array 50 of FIG. 5.

The process of calculating the area under a ROC curve generated based on the layer thickness assigned to a specific location of the eye 10 for a plurality, and optionally many different subjects, and assigning that calculated area to a corresponding entry in the ROC array 50 is repeated for each specific location 27 in the region of interest 24. The accuracy of using the retinal thickness, alone, as the test for glaucoma is measured by the area under the ROC curve. Thus, determining the value of the area under the ROC curve for each entry of the ROC array 50 provides an indication how accurate of a predictor the retinal thickness at each specific location 27 is for determining whether a subj ect has glaucoma.

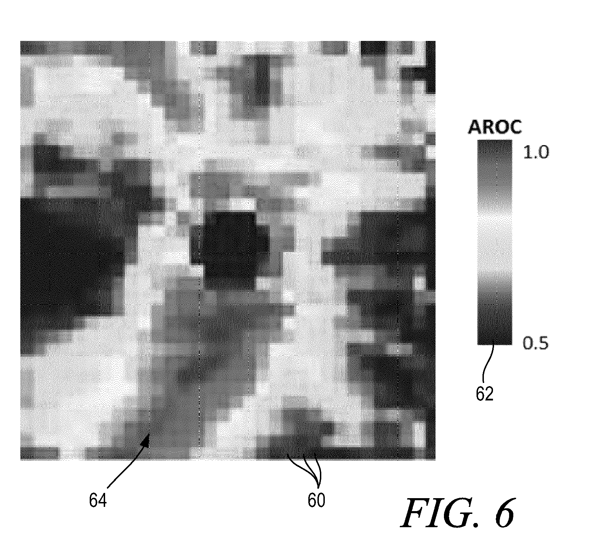

To provide a readily-observable, graphical representation of the accuracy of the retinal thickness in testing for glaucoma at each specific location 27 in the region of interest 24, a ROC map such as that appearing in FIG. 6 can be generated. Each value, or range of values for Rₙₘ of the area under the ROC curve for each of the spatial locations 27 can be assigned a color code or other visible indicator. The numeric values for Rₙₘ can be replaced by their respective color code or other visible indication, allowing specific locations 27 or collections of specific locations 27 in the region of interest 24 to be readily observable as either good or poor indicators of a condition such as glaucoma. Since the cells in the ROC array 50 (FIG. 5) can be arranged to correspond to the arrangement of spatial locations 27 (FIG. 2) in the grid 28, an observer can visually ascertain spatial locations 27 within the eye 10 that have historically provided a more-definitive indication whether a subj ect has glaucoma or not. In other words, the color representing the area under the ROC curve for each spatial location 27 in FIG. 6 determined using the historical clinical data as described herein can be arranged in a similar, and optionally the same arrangement as the spatial locations 27 of the region of interest 24 within the eye 10 as shown in FIG. 2. Analysis of a current subject's thickness array 44, which can also optionally be color coded, for purposes of the diagnoses can be limited primarily to the measurements taken for the specific locations 27 that have proven to be more definitive predictors.

An example of the color coding is shown in FIG. 6, which provides a visible representation of a ROC array that is larger (i.e., a greater number of columns and rows) than the ROC array 50 in FIG. 5. As shown, each pixel 60 is filled with a color representing the underlying Rₙₘ value for a corresponding cell in the ROC array. A legend 62 relates each different color to a different Rₙₘ value corresponding to the area under the ROC curve for the different spatial locations 27. For the embodiment shown in FIG. 6, the legend represents a range of areas under the ROC curve ("AROC") from 0.5 to 1.0. An inferior arcuate region 64 in the present example, which corresponds to similarly-located spatial locations 27, is shown as a region where meaningful thickness measurements are located for the purpose of diagnosing glaucoma.

The values Rₙₘ in the ROC array 50 serve to identify which of the corresponding spatial locations 27 are most meaningful for generalized consideration. In other words, based on the Rₙₘ values and the optional color code or other visible indicator, an observer can ascertain which of the spatial locations 27 of the retina are most likely to yield definitive information used in the diagnosis of glaucoma. For the illustrative embodiment of the visible indicator provided to the ROC array 50 in FIG. 6, it can be seen that a distinctive pattern exists in and around the inferior arcuate region 64, a somewhat less distinctive region exists around the superior arcuate region, and the region immediately surrounding the optic disc can also optionally be considered notable as being a possible predictor of glaucoma based solely on the retinal thickness at those spatial locations 27. The ROC map, therefore, suggests that clinicians should concentrate diagnostic efforts in such locations (although they will not necessarily limit to those areas).

The ability of the spatial locations to predict whether a patient has a medical condition such as glaucoma can optionally be utilized by the processor provided to the computer to emphasize the spatial locations most likely to be a reliable predictor. Reference points such as the fovea, optic disc, etc... are generally located and placed at the center of, or can be positioned at any arbitrary fixed and known location as a reference within the OCT scanning field or grid 28 (FIG. 2). The scan then proceeds to encompass the entire region of interest, and data collected throughout the entire region is utilized in diagnosing the patient as either having or not having glaucoma. According to alternate embodiments, the computer processor can be programmed to consider the values Rₙₘ in the ROC array 50 to identify the spatial locations that are relatively reliable predictors of glaucoma compared to other spatial locations. To accomplish this, the computer 25 can optionally control the scan path established by the transmitter 11 (FIG. 1) to scan along a predetermined, closed-loop path (e.g., circular, elliptical, etc...) covering the spatial locations indicated by the values Rₙₘ in the ROC array 50 as being reliable predictors of glaucoma. Thus, the scan path can be established with a suitable size and/or shape to encompass those spatial locations that are reliable predictors. According to other embodiments, the scan path can include all spatial locations 27, spatial locations in a path that is not closed loop, or an arbitrary scan path including only regions of desired spatial locations 27 within the region of interest, but the computer 25 can be programmed to limit post-capture processing steps to be performed only on those spatial locations considered to be reliable predictors of glaucoma based on the values Rₙₘ in the ROC array 50. Regardless of how the data collected is limited to the spatial locations considered to be reliable predictor, such a step limits the data that must be considered and analyzed by a clinician attempting to make a diagnosis of a medical condition, and will ultimately lead to improved accuracy of disease detection in terms of the resulting sensitivity and specificity characteristics.

The spatial locations considered to be reliable predictors can be distinguished from other spatial locations and selected based on any suitable criteria. For example, the scan path can be established such that the average value Rₙₘ in the ROC array 50 corresponding to the spatial locations considered to be reliable predictors exceeds a predetermined threshold value (e.g., greater than an arbitrarily selected value specific to the diagnosis to be made such as 0.8 or 0.9). According to alternate embodiments, the spatial locations can optionally be considered to be reliable predictors of glaucoma if their corresponding values Rₙₘ in the ROC array 50 exceed a predetermined threshold. In yet another embodiment, an optimization technique, such as path search, over the ROC map can be applied to maximize the integrated ROC area under the curve values encountered over the path, thereby determining an optimal scan path.

Although the examples above involved the interpretation of retinal thickness within a region of interest in the eye to assist in the diagnosis of glaucoma, it is to be understood that the present disclosure is not so limited. Instead, the concepts discussed herein are applicable to analyzing data in any demonstrable spatial patterns within a region of interest anywhere on a subject for any diagnostic or other effort. Additionally, the measured quantities used for diagnosis are not necessarily physical properties such as the dimension of a tissue layer. Other optical, acoustic, electrical, chemical, etc... properties such as an attenuation coefficient or integrated attenuation of tissue, for example, can be utilized and compared to a ROC analysis of historical data for diagnostic purposes as described herein.

Illustrative embodiments have been described, hereinabove. It will be apparent to those skilled in the art that the above devices and methods may incorporate changes and modifications without departing from the general scope of this invention. It is intended to include all such modifications and alterations within the scope of the present invention. Furthermore, to the extent that the term "includes" is used in either the detailed description or the claims, such term is intended to be inclusive in a manner similar to the term "comprising" as "comprising" is interpreted when employed as a transitional word in a claim.

## Claims

1. A system (7) for presenting experimentally-determined data relating to a region of interest on a medical patient, the system comprising:
a non-transitory, computer-readable medium storing a receiver operating characteristic analysis of historical data for a region of interest (24) on each of a plurality of different subjects, wherein the region of interest (24) on each of the plurality of different subjects corresponds to the region of interest on the medical patient;
a processor programmed to perform a comparison of the experimentally-determined data to the receiver operating characteristic analysis of the historical data stored by the computer-readable medium and identify the experimentally-determined data to be utilized to reach an at least preliminary conclusion regarding the medical patient based on the experimentally-determined data; and
a display device (25) operatively connected to the processor to present a visible display graphically depicting a result of the comparison performed by the processor.

2. The system of claim 1, wherein the computer-readable medium stores the receiver operating characteristic analysis of tissue thickness data collected within the region of interest in each of the plurality of different subj ects, wherein, in particular, the tissue thickness is measured using at least one of either or both of an A-scan and a B-Scan and/or wherein in particular the tissue thickness data of each subject is normalized, for example based on the size and/or position of at least one of: the optic disc, fovea, blood vessels, nerve fiber layer arcuates, other portion of an eye (10), and any combination thereof and/or based on the size and/or position of landmark features.

3. The system of claim 1 or 2, wherein the processor is programmed to utilize a subset of the receiver operating characteristic analysis including historical data of the plurality of different subjects with a desired demographic in common with the medical patient.

4. The system of any one of the preceding claims, wherein said comparison performed by the processor comprises a comparison of the experimentally-determined data to the receiver operating characteristic analysis of the historical data for a left eye (10) of the plurality of different subjects to the experimentally-determined data for the left eye of the medical patient.

5. The system of any one of the preceding claims, wherein the processor is programmed to compare the experimentally-determined data to the receiver operating characteristic analysis of the historical data that is weighted based on a number of the plurality of different subjects that contributed to the experimentally-determined data at each of a plurality of spatial locations (27) within the region of interest, wherein, in particular, the plurality of spatial locations (27) are stored in a respective array (36) with entries corresponding to a grid (28), wherein, for example, a respective array (36) represents the intensity values of an A-scan for a given tissue depth, the respective array is a thickness array (44) storing the layer thickness calculated at each of the different spatial locations (27) determined from the B-scan and/or the respective array (50) includes data generated as a result of the receiver operating characteristics analysis.

6. The system of any one of the preceding claims, wherein the processor is programmed to establish a likelihood that the experimentally-determined data by itself at a plurality of different spatial locations (27) is an accurate predictor of whether the medical patient has a medical condition based at least in part on the receiver operating characteristic analysis of the historical data at the plurality of different spatial locations of the plurality of different subjects.

7. The system of claim 6, wherein the processor is programmed to generate a display graphically representing the likelihood as an image lacking alphanumeric characters.

8. A method of graphically depicting spatial locations within a region of interest that are useful predictors of a medical condition, the method comprising:
Receiving, by a computer processor, clinical data specific to the spatial locations for a plurality of different patients obtained during medical examinations of those patients, wherein the plurality of different patients comprise a plurality of positive patients who have been independently confirmed to have the medical condition and a plurality of negative patients who have been independently confirmed as not having the medical condition;
utilizing the computer processor for conducting a receiver operating characteristic analysis of the clinical data; and
generating, by the computer processor, a graphic representation of a result of the receiver operating characteristic analysis for each of the spatial locations, wherein the graphic representation comprises an arrangement of each result of the receiver operating characteristic analysis that reflects the respective arrangement of each of the plurality of different locations of the patients where the clinical data was collected.

9. The method of claim 8, wherein the clinical data comprises a thickness of ocular tissue at the plurality of different locations of an eye of the patients.

10. The method of claims 8 or 9 further comprising:
capturing current clinical data from the different spatial locations on a current patient who is undergoing medical examination to determine whether the current patient is afflicted with the medical condition;
generating a graphical display comprising an arrangement of the current clinical data captured from the different spatial locations on a current patient, wherein the current clinical data is arranged in an arrangement that reflects the arrangement of the result of the receiver operating characteristic analysis for each spatial location.

11. The method of any one of the claims 8 to 10, wherein said result of the receiver operating characteristic analysis for the spatial locations comprises values reflecting an area under a receiver-operating-characteristic curve for the plurality of different locations.

12. The method of claim 11, wherein the values reflecting an area under a receiver-operating-characteristic curve included as the result of the receiver operating characteristic analysis are represented by a color appearing in locations corresponding to the plurality of different locations where the clinical data was collected.

13. The method of any one of the claims 8 to 12 further comprising filtering the clinical data of the plurality of different patients based on age and limiting the receiver operating characteristic analysis of the clinical data to the receiver operating characteristic analysis of the clinical data collected from a subset of the plurality of different subjects with an age satisfying a filter age criterion.

14. The method of any one of the claims 8 to 13, wherein the graphic display comprises an image that is devoid of alphanumeric characters.

15. The method of any one of the claims 8 to 14, wherein the subset of the spatial locations comprises locations within a region extending from a cornea to a retina of an eye of the plurality of different patients.
